Europäisches Patentamt

**European Patent Office**

Office européen des brevets

(19)

(11) Numéro de publication: **0 194 170**
**B1**

# (12) FASCICULE DE BREVET EUROPEEN

(45) Date de publication du fascicule du brevet:
**12.04.89**

(51) Int. Cl.⁴: **A 23 K 1/16** // A23L1/305

(21) Numéro de dépôt: **86400196.1**

(22) Date de dépôt: **30.01.86**

(54) Nouvelles compositions pour l'alimentation des animaux.

(30) Priorité: **01.02.85 FR 8501418**

(43) Date de publication de la demande:
**10.09.86 Bulletin 86/37**

(45) Mention de la délivrance du brevet:
**12.04.89 Bulletin 89/15**

(84) Etats contractants désignés:
**AT BE CH DE FR GB IT LI LU NL SE**

(73) Titulaire: **RHONE-POULENC NUTRITION ANIMALE, Rue Marcel Lingot, F-03600 Commentry (FR)**

(72) Inventeur: **Bercovici, Daniel, 13 rue Colonel Driant, F-06100 Nice (FR)**
Inventeur: **Gaertner, Hubert, 62 rue Jules Isaac, F-13009 Marseille (FR)**
Inventeur: **Puigserver, Antoine, 14 rue Maréchal Fayolle, F-13004 Marseille (FR)**

(74) Mandataire: **Pilard, Jacques et al, RHONE-POULENC RECHERCHES Service Brevets Pharma 25, Quai Paul Doumer, F-92408 Courbevoie Cedex (FR)**

(56) Documents cités:
JOURNAL OF AGRICULTURAL AND FOOD CHEMISTRY, vol. 32, no. 6, novembre-décembre 1984, pages 1371-1376, American Chemical Society, Washington, US; H.F. GAERTNER et al.: "Covalent attachment of Poly(L-methionine) to food proteins for nutritional and functional improvement"
NUTRITION REPORTS INTERNATIONAL, vol. 22, no. 5, novembre 1980, pages 707-715; C.W. NEWMAN et al.: "Poly-1-lysine, a nutritional source of lysine"
CHEMICAL ABSTRACTS, vol. 101, no. 23, 3 décembre 1984, page 532, abrégé no. 209654q, Columbus, Ohio, US; K.R. DABROWSKI: "Lysine requirement for common carp (Cyprinus carpio L.) fry" & COLLOQ.

(56) Documents cités: (suite)
INRA 1983, 16(Metab. Nutr. Azotes, v2), 473-6
JOURNAL OF AGRICULTURAL AND FOOD CHEMISTRY, vol. 26, no. 2, mars-avril 1978, pages 341-345, American Chemical Society, Washington, US; H.E. AMOS et al.: "In vitro rumen microbiol stability and in vivo availability of polymerized L-lysine-HCl"

## Description

La présente invention concerne de nouvelles compositions pour l'alimentation des animaux contenant de la poly méthionyl-poly lysine, la méthionine et la lysine étant sous forme L.

La lysine et la méthionine sont des aminoacides essentiels qui favorisent la croissance des animaux et il est souvent nécessaire de compléter les rations alimentaires par des compositions contenant ces aminoacides qui permettent la libération de l'aminoacide dans l'organisme. Dans le cas particulier des ruminants, il est important, pour que les aminoacides puissent exercer leur action bénéfique, que les compositions soient stables dans le rumen et libèrent les aminoacides ou les oligopeptides assimilables dans l'intestin.

C. W. Newman et coll., Nutrition Reports Int., 22 (5) 705—715 (1980) enseignent que la poly L lysine peut être utilisée pour compenser la carence en lysine chez le rat, la poly L lysine ayant une efficacité équivalente à celle du chlorhydrate de L lysine.

K. R. Dubrowski, Chem. Abstr., 101, 209654q (1984) enseigne l'utilisation de la poly L lysine comme facteur de croissance chez la carpe, la poly L lysine donnant des résultats inférieurs à ceux qui sont obtenus avec la D, L lysine ou la L lysine.

Enfin H. F. Gaertner et A. J. Puigserver, J. Agr. Food Chem., 32, 1371—1376 (1984) enseignent le couplage de poly méthionine aux restes lysines des protéines alimentaires.

Il a maintenant été trouvé, et c'est ce qui fait l'objet de la présente invention, que les aliments pour animaux peuvent être supplémentés par des poly méthionyl-poly L lysine qui conservent les propriétés des aminoacides.

Les poly L méthionyl-poly L lysine utilisables dans les compositions selon l'invention ont un poids moléculaire compris entre 1000 et 1 000 000 daltons et de préférence entre 20 000 et 200 000 daltons et le rapport méthionine/lysine est compris entre 0,4 et 4.

La poly L méthionyl-poly L lysine peut être préparée par action du N-carboxyanhydride de la méthionine sur la poly lysine selon la méthode décrite par Gaertner et Puigserver (1984) pour préparer des polyméthionyl protéinés.

La réaction du N-carboxyanhydride de la méthionine la poly lysine peut être effectuée à un pH compris entre 6 et 11 en opérant en milieu tamponné et en utilisant un excès molaire de N-carboxyanhydride de la méthionine par rapport aux résidus de lysine.

Généralement la réaction s'effectue à une température voisine de 4°C.

L'utilisation du N-carboxyanhydride de la méthionine à un pH voisine de 6, par exemple 6, 5, favorise la polymérisation de cet aminoacide sur les groupements aminés du polymère. Dans ce cas, on utilise généralement un excès molaire de N-carboxyanhydride de la méthionine compris entre 1 et 5 par rapport aux résidus de lysine.

En opérant à un pH voisin de 11, par exemple 10, 2, il est préférable de procéder par additions successives du N-carboxyanhydride de la méthionine pour obtenir une meilleure répartition des résidus de méthionine greffés. Entre chaque addition du réactif, en utilisant à chaque fois un excès molaire de réactif compris entre 1 et 1,5 par rapport aux résidus lysine, le mélange réactionnel est amené à température ambiante et son pH est abaissé au voisinage de 3—4 afin de permettre la décarboxylation du carbamate intermédiaire et une nouvelle fixation du réactif sur les groupements aminés libres.

L'excès de N-carboxyanhydride de la méthionine par rapport aux groupements aminés libres de la poly lysine permet de déterminer la proportion relative de méthionine et de lysine dans le produit fini. Le rapport méthionine/lysine est compris entre 0,4 et 4.

La poly méthionyl-poly lysine est généralement isolée après lyophilisation de sa solution aqueuse préalablement dialysée.

Les poly lysine peuvent être obtenues par polymérisation du N-carboxyanhydride de la εN-carbobenzoxy lysine dans un solvant organique tel que le dioxanne en présence d'une base aprotique, telle que le méthylate de sodium ou la diéthylamine, qui joue le rôle d'initiateur en opérant à une température voisine de 20°C. Le contrôle du rapport entre l'initiateur et le dérivé de la lysine mis en œuvre permet de faire varier la taille des polymères formés.

L'élimination des groupement protecteurs N-carbobenzoxy peut être réalisée selon la technique de Ben Ishai et Berger (1959) au moyen d'acide bromhydrique dans l'acide acétique.

La poly lysine précipitée est séparée puis purifiée par dialyse après solubilisation dans l'eau. La poly lysine est séparée par lyophilisation de sa solution aqueuse.

Sur la polymère ainsi obtenu, il est possible de contrôler l'élimination du groupement protecteur (tranformation en acide α-amino ε-hydroxycarproïque, hydrolyse par la trypsine).

Le poids moléculaire de la poly lysine obtenue peut être déterminé à partir du coefficient de sédimentation mesuré par ultra-centrifugation analytique ou par la mesure de la viscosité spécifique.

Les N-carboxyanhydrides de la N-carbobenzoxy lysine et de la méthionine utilisé pour la préparation des polymères entrant dans les compositions selon l'invention peuvent être préparés selon la méthode décrite par Hirschman et coll (1971) par action du phosgène anhydre sur une suspension de l'aminoacide dans un solvant organique inerte tel que le tétrahydrofuranne anhydre en opérant à une température comprise entre 30 et 60°C et de préférence voisine de 45°C.

Les exemples suivants illustrent la préparation des polymères selon l'invention.

## Exemple 1

Le N-carboxyanhydride de la N-carbobenzoxy L lysine polymérise en présence d'un initiateur (méthylate de sodium) en opérant dans le dioxanne à 20°C. La disparition du monomère du milieu réactionnel permet de suivre sa polymérisation. La polymérisation est achevée au bout de 75 heures. Le polymère obtenu se présente sous forme d'un produit visqueux.

Le polymère est traité par de l'acide acétique saturé d'acide bromhydrique pendant 30 minutes.

Le précipité formé est lavé par l'éther éthylique et séché. Après solubilisation dans l'eau suivie de dialyse, la poly L lysine obtenue est séparée de sa solution par lyophilisation. Le poids moléculaire est voisin de 290 000 daltons.

La désamination de la poly L lysine permet de vérifier la présence des groupements aminés libres. Après hydrolyse acide l'analyse révèle un pic unique correspondant à la présence d'acide α-amino ε-hydroxy-caproïque.

La N-carboxyanhydride de la N-carbobenzoxy L lysine est obtenue par barbotage de phosgène anhydre dans une suspension de εN-carbobenzoxy L lysine dans le tétrahydrofuranne anhydre à 45°C. La réaction terminée, un barbotage d'azote anhydre permet d'éliminer le phosgène en excès.

Après évaporation du solvant, le produit est dissous dans de l'acétate d'éthyle. Le solvant est évaporé et le résidu est repris par de l'acétate d'éthyle. L'opération est répétée 2 fois. Le N-carboxyanhydride de la N-carbobenzoxy L lysine cristallise par addition lente de n-hexane dans sa solution dans l'acétate d'éthyle jusqu'à un rapport 1–1 (en volumes).

Les cristaux obtenus sont lavés par un mélange hexaneacétate d'éthyle (9–1 en volume); ils fondent à 97°C.

Exemple 2

La poly L lysine en solution à 5 mg/cm³ de tampon phosphate de sodium (100 mM) à pH 6,5 contenant 150 mM de chlorure de sodium est maintenue à 4°C. En agitant vigoureusement et en maintenant le pH constant par addition de soude 0,1 N, on ajoute 1 mole de N-carboxyanhydride de la méthionine par résidu de lysine.

Après une nuit à 4°C, le produit est dialysé contre de l'eau pendant 3 jours puis la solution est hyophilisée.

On obtient ainsi une poly L méthionyl-poly L lysine dans laquelle le rapport méthionine/lysine: 0,55 et contenant 27 % de résidus de lysine modifiée.

Le rendement de fixation est voisin de 80 %.

Le N-carboxyanhydride de la L méthionine qui se présente sous forme d'un produit huileux est obtenu dans les conditions décrites précédemment pour l'obtention du N-carboxyanhydride de la N-carbobenzoxy L lysine.

Exemple 3

En opérant comme dans l'exemple 2 mais en utilisant 3 moles de N-carboxyanhydride de la L méthionine par résidu de lysine, on obtient une poly L méthionyl-poly L lysine dans laquelle le rapport méthionine/lysine = 1,90 et contenant 49 % de résidus lysine modifiés.

Exemple 4

En opérant comme l'exemple 2 mais en utilisant 5 moles de N-carboxyanhydride de la L méthionine par résidu lysine, on obtient une poly L méthionyl-poly L lysine dans laquelle la rapport méthionine/lysine = 3,40 et contenant 70 % de résidus lysine modifiés.

Exemple 5

La poly L lysine est mise en solution à 4°C dans une solution tamponnée de bicarbonate de sodium (100 mM) à pH 10,2 contenant 150 mM de chlorure de sodium. On ajoute 1 mole de N-carboxyanhydride de la L méthionine par résidu lysine. On laisse la température monter à 20°C et le pH du mélange réactionnel est ajusté à pH 3–4 pendant 10 minutes. Après refroidissement à 4°C, le produit est dialysé contre de l'eau distillée pendant 3 jours, puis la solution est lyophilisée.

On obtient ainsi une poly L méthionyl-poly L lysine dans laquelle le rapport méthionine/lysine = 0,45 et contenant 45 % de résidus de lysine modifiés.

Exemple 6

On opérant comme dans l'exemple 5 mais en ajoutant 1,2 mole de N-carboxyanhydride de la L méthionine par résidu lysine, on obtient une poly L méthionyl-poly L lysine dans laquelle le rapport méthionine/lysine = 0,53 et contenant 50 % de résidus lysine modifiés.

Exemple 7

En opérant comme dans l'exemple 6 mais en ajoutant 3 fois 1 mole de N-carboxyanhydride de la L méthionine par résidu lysine, on obtient une poly L méthionyl-poly L lysine dans laquelle le rapport méthionine/lysine = 2,10 et contenant 66 % de résidu lysine modifiés.

La disponibilité de la lysine et de la méthionine au sein des polymères peut être mise en évidence in vitro par digestions successives au moyen des enzymes présentes dans le tractus digestif telles que la pepsine, le suc pancréatique et l'aminopeptidase intestinale.

Dans le tableau I sont indiquées les quantités relatives de lysine et de méthionine libérées au cours de la digestion:

Tableau I

| Exemples | Methionine/ lysine | Methionine liberée % | Lysine liberée % |
| --- | --- | --- | --- |
| 2 | 0,55 | 45 | 44 |
| 3 | 1,90 | 63 | 53 |
| 4 | 3,40 | 18 | 5 |
| 5 | 0,45 | 56 | 54 |
| 6 | 0,53 | 54 | 60 |
| 7 | 2,10 | 2 | 5 |

Suivant le type de polymère utilisé, les disponibilités des deux aminoacides sont très variables.

Généralement, les produits obtenus par polymérisation à pH 6,5 qui ont une teneur élativement élevée en méthionine (exemple 3) sont facilement hydrolysés, alors que les produits obtenus à pH = 10,2 qui sont caractérisés par une proportion importante de résidus lysine modifiés (exemple 7) sont moins accessibles aux enzymes protéolytiques. Par ailleurs, les polymères obtenus à pH 6,5 qui comportent une proportion importante de résidus lysine modifiés sont plus facilement hydrolysés que ceux qui sont obtenus à pH 10,2 par additions successives du N-carboxyanhydride de la méthionine.

Généralement, une disponibilité satisfaisante est obtenue lorsque le rapport méthionine/lysine est inférieur à 2 et de préférence voisin de 1, ce taux pouvant varier en fonction du procédé d'obtention de la poly méthionyl-poly lysine.

La valeur nutritionnelle des poly méthionyl-poly lysine utilisées dans les compositions selon la présente invention a été mise en évidence chez le rat recevant un régime de base carencé en lysine et en méthionine. Par exemple, en utilisant la poly L lysine et une poly L méthionyl-poly L lysine dans laquelle le rapport méthionine/lysine est voisin de 1 et contenant 30 % de résidus lysine modifiés, les résultats qui ont été obtenus sont rassemblés dans le tableau II.

Tableau II

| Regime Alimentaire | Nourriture Ingeree g/14 Jours | Proteines Ingerees g/14 Jours | Gain de Poids g/14 Jours | Coefficient D'efficacite Proteique |
|---|---|---|---|---|
| UAR | 208,9+27,5 | 20,89+2,75 | 41,30+5,20 | 1,98+0,12 |
| UAR+ Met +lys (libres) | 189,6+28,1 | 20,67+3,06 | 52,15+13,00 | 2,67+0,35 |
| UAR+ Met (libre) +poly L lysine | 227,9+26,9 | 25,30+2,99 | 72,30+ 8,70 | 2,86+0,14 |
| UAR+ Poly L Methio-nyl Poly L Lysine | 197,1+23,0 | 21,90+2,56 | 55,60+10,60 | 2,52+0,25 |

De ce tableau, il ressort que la supplémentation du régime de base en lysine et en méthionine libres améliore significativement la croissance des animaux et que la croissance des animaux nourris par un régime supplémenté par la poly L méthionyl-poly L lysine n'est pas significativement différente de celle des animaux dont le régime est supplémenté en acides aminés libres. Par ailleurs, la supplémentation en lysine sous forme de poly L lysine est significativement plus efficace que sous forme libre.

Par ailleurs, les poly méthionyl-poly lysine selon la présente invention présentent la propirété remarquable d'être soluble dans le rumen des ruminants en résistant à la dégradation bactérienne. Par exemple, la poly L méthionyl-poly L lysine (0,5 g) peut être introduite dans des sachets en nylon dont le vide de maille est de $300 \times 300\mu m$. Les sachets sont mis à incuber pendant 48 heures dans le rumen de brebis fistulées. Les sachets sont ensuite récupérés et lavés. La substance active restante est quantitativement déterminée selon une méthode appropriée. Les quantités de L lysine et L méthionine retrouvées dans les sachets en nylon représentent respectivement 80 et 60% des teneurs initiales. Lorsque la L lysine et la L méthionine sont ingérées sous forme libre seule une faible proportion de ces aminoacides échappe à la fermentation microbienne.

Les poly méthionyl-poly lysine selon l'invention constituent donc des adjuvants de choix pour l'alimentation des ruminants.

La présente invention concerne les compositions pour l'alimentation des animaux qui contiennent une quantité suffisante de poly méthionyl-poly lysine.

Les poly méthionyl-poly lysine peuvent être répartis en dispersion uniforme dans les aliments composés complets.

Ils peuvent être répartis dans les aliments complémentaires, le plus souvent avec d'autres additifs tels que vitamines et sels minéraux. Ces aliments complémentaires peuvent être soit mélangés à la ration, soit consommés tels quels et ils représentent habituellement 5 à 20% de la ration.

Les «prémixes», utilisés pour la préparation des aliments complémentaires ou des rations complètes, contiennent généralement de 1 à 20% de poly méthionyl-poly lysine. Ils constituent un intermédiaire commode facilitant la répartition uniforme de l'adjuvant dans les aliments.

Les «prémixes» eux-mêmes sont généralement obtenus à partir de concentrés qui contiennent de 99,9 à 20% de poly méthionyl-poly lysine additionné de dénaturants comestibles tels que colorants alimentaires, aromatisants, agents dispersants ou évitant l'agglomération et charges alimentaires.

Les concentrés et prémixes sont généralement pulvérulents. Les aliments complémentaires et les aliments composés complets peuvent être soit pulvérulents, soit sous forme de granulés préparés selon les techniques habituelles.

**Revendication**

Composition pour l'alimentation des animaux, et plus particulièrement des ruminants caractérisée en ce qu'elle contient une quantité suffisante d'une poly L méthionyl-poly L lysine dont le poids moléculaire est compris entre 1000 et 1 000 000 et dans laquelle le rapport méthionine/lysine est comprise entre 0,4 et 4.

## Claim

Composition for feeding animals, and more especially ruminants, characterized in that it contains a sufficient amount of a poly-L-methionyl-poly-L-lysine the molecular weight of which is between 1000 and 1000000 and in which the methionine/lysine ratio is between 0.4 and 4.

## Patentanspruch

Futtermittelmischung für Tiere und speziell für Wiederkäuer, dadurch gekennzeichnet, daß sie eine ausreichende Menge eines Poly-L-methionyl-L-lysins enthält, dessen Molekulargewicht zwischen 1000 und 1000000 liegt und worin das Verhältnis Methionin/Lysin zwischen 0,4 und 4 liegt.